# EUROPEAN PATENT APPLICATION

(11) **EP 2 939 595 A1**
(43) Date of publication of application: **04.11.2015**
(21) Application number: 14166267.6
(22) Date of filing: 28.04.2014
(51) Int. Cl.: A61B 5/087, A61B 5/113, A61B 5/00

(54) **A method and system for determining a movement of an object**

(71) Applicant: Advanced Digital Broadcast S.A., 1292 Chambesy (CH)
(72) Inventor: Jankowski, Bartosz, 65-119 Zielona Gora (PL); Micewicz, Jaros aw, 65-119 Zielona Gora (PL)
(74) Representative: Pawlowski, Adam

(57) **Abstract**

A method for determining a movement of an object (100) in an optical axis (A) of a camera (101), comprising the steps of: placing (201) a camera with the optical axis directed towards the object; activating (202) camera continuous autofocus functionality; monitoring (203) autofocus parameter pattern change; and determining (204) the movement of the object depending on the autofocus parameter pattern change.

## Description

### TECHNICAL FIELD

The present invention relates to a method and system for determining small movements of an object by a passive system, which can be used e.g. to monitor breathing of a person during sleep.

### BACKGROUND

Deficiencies and irregularities in breathing can have very serious consequences on health. This is a case especially during sleeping, which normally occurs in lying position. A sleeping person cannot knowingly observe his irregular breathing and any problems related to it, and therefore a quick reaction is often not possible. Also a person who is aware of his problems related to breathing, but does not have a companion who is monitoring his sleep and who would spot abnormal breathing patterns, lacks a protection that would allow for quick reaction in case of an emergency situation.

There are known a number of approaches to methods for monitoring of breathing.

A US patent application US20100061596 discloses a method for determining similarity with a portion of a psychological motion, which utilizes images of infant during sleep. The method includes steps of comparing two images of the object and determining a level of similarity between them. Then, the level of similarity is correlated with a portion of physiological information.

Another US patent application US20120253178 discloses a system and method for triggering an imaging process based on non-periodicity in breathing. The signals concerning breathing are gathered and analyzed with respect to any non-periodicity. In case of such non-periodicity, an imaging process is initiated. System uses a marker block, the movement of which is monitored by a processor with help of a camera. The processor is configured to continuously process image signals and determine the position of the marker block, therefore determining the breathing amplitude of the patient in substantially real time.

An international application WO2013093731 discloses a method and a system for video processing for monitoring breathing of a person in poor lighting conditions. It takes into account such factors as shadows and noise, and provides computer implemented approach to help reduce their adverse effects.

A drawback of the above methods and systems is that they require picture analyses to recognize patterns of changes. They require a significant computational power, which in turn leads to higher energy consumption, costs of manufacturing and overall complexity.

A US patent US5825293 presents an apparatus and method for monitoring breathing magnetically. The patient wears a sensing element coupled with a detector. The sensing element is placed in a manner allowing for detection of its movement by the detector, for instance it can include a permanent magnet. The detector is then a magnetic detector configured to monitor magnetic field caused by the magnet and provide an alarm signal when a measured rate of variation is detected to be outside of predetermined rate limit.

A US patent US7575554 discloses a breathing monitoring device for monitoring movement of, for example, chest. The device comprises a housing, which is placed above the chest of the patient. The device is equipped with a displacement amount determination unit and a respiration level determination unit.

A US patent application US20130289431 shows an apparatus and method for breathing pattern determination using a non-contact microphone. It utilizes a raw signal indicative of air-flow sounds of the respiration, which is analyzed to determine sets of parameters of respiration.

The above methods and systems require additional devices or elements that have to be in contact or in close vicinity to the user. They limit therefore his spatial maneuverability and are prone to accidental dislocation or damaging.

It is therefore the aim of the present invention to provide further improvements to systems and methods for monitoring of movement of object in an optical axis of a camera, which can be used e.g. to monitor breathing patterns, that would reduce complexity and require less processing power and would not necessitate additional elements attached to or placed near a user.

### SUMMARY

There is presented a method for determining a movement of an object in an optical axis (A) of a camera, comprising the steps of: placing a camera with the optical axis directed towards the object; activating camera continuous autofocus functionality; monitoring autofocus parameter pattern change; and determining the movement of the object depending on the autofocus parameter pattern change.

Preferably, the method further comprises the steps of: analyzing the pattern of the movement of the object; and outputting an alarm signal upon recognizing an abnormal pattern.

Preferably, the method comprises comparing the determined parameter pattern with model parameter patterns from a database.

There is also presented use of the above-presented method to monitor the breathing pattern of a person.

There is also presented a computer program comprising program code means for performing steps of the method when said program is run on a computer, as well as a computer readable medium storing computer-executable instructions performing steps of the method when executed on a computer

There is further presented a system for determining a movement of an object in an optical axis (A) of a camera, comprising: a camera placed with the optical axis (A) directed towards the object; and a computer system connected to the camera via a camera interface and comprising a controller configured to: activate camera continuous autofocus functionality; monitor autofocus parameter pattern change; and determine the movement of the object depending on the autofocus parameter pattern change.

Preferably, the system further comprises an alarm interface, wherein the controller is configured to analyze the pattern of the movement of the object and to output an alarm signal via the alarm interface upon recognizing an abnormal pattern.

Preferably, the system further comprises a database of model parameter patterns, wherein the controller is configured to compare the determined parameter pattern with model parameter patterns from the database.

### BRIEF DESCRIPTION OF DRAWINGS

The present invention is shown by means of exemplary embodiments on a drawing, in which:
Fig. 1 illustrates an exemplary arrangement of the camera with respect to the moving object;
Fig. 2 shows the steps of a method for monitoring a moving object;
Fig. 3 shows a system for monitoring a moving object;

### NOTATION AND NOMENCLATURE

Some portions of the detailed description which follows are presented in terms of data processing procedures, steps or other symbolic representations of operations on data bits that can be performed on computer memory. Therefore, a computer executes such logical steps thus requiring physical manipulations of physical quantities.

Usually these quantities take the form of electrical or magnetic signals capable of being stored, transferred, combined, compared, and otherwise manipulated in a computer system. For reasons of common usage, these signals are referred to as bits, packets, messages, values, elements, symbols, characters, terms, numbers, or the like.

Additionally, all of these and similar terms are to be associated with the appropriate physical quantities and are merely convenient labels applied to these quantities. Terms such as "processing" or "creating" or "transferring" or "executing" or "determining" or "detecting" or "obtaining" or "selecting" or "calculating" or "generating" or the like, refer to the action and processes of a computer system that manipulates and transforms data represented as physical (electronic) quantities within the computer's registers and memories into other data similarly represented as physical quantities within the memories or registers or other such information storage.

### DESCRIPTION OF EXAMPLE EMBODIMENTS

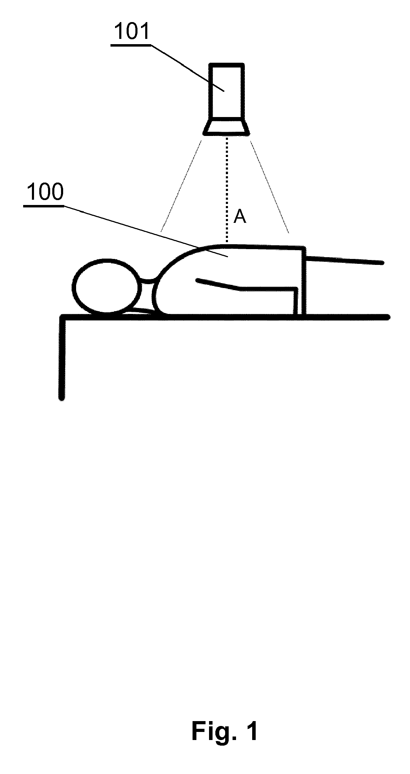

Fig. 1 shows a camera 101 placed above a moving object, such that the object movement occurs in the optical axis A of the camera. In the present example, the moving object is a person, e.g. during sleep, whose check moves back and forward with respect to the camera.

The camera 101 is used to obtain information about object movement without physical contact. The system utilizes a typical passive autofocus function of the camera. The autofocus is concentrated on the area of a chest of the person, and is constantly following the changes in the distance between the chest and the camera through focus readjustment. The changes can be measured along the optical axis A of the camera, which is directed to a selected point on the chest. They can be also derived from points lying beyond the optical axis A, but still captured by the camera. Monitoring of the autofocus parameters can concern for example monitoring autofocus oscillations as a time function. The camera thus observes the person or a quilt (if present) and regulates autofocus in the rhythm of the breath. The autofocus pattern therefore represents the breathing pattern.

The camera 101 can be any digital camera provided with passive continuous autofocus function. In some conditions, the persons clothing (e.g. pyjamas) have some contrasting element to improve autofocus functionality (e.g. in form of a contrasting, black sticker attached to persons clothing). Autofocus function is a well-known tool, and is thoroughly described in literature known to persons skilled in the art.

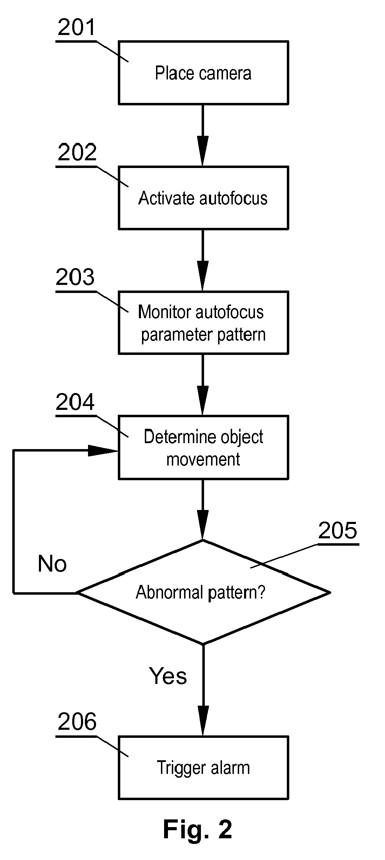

Fig. 2 presents a method for monitoring a moving object. Firstly, the camera is positioned in step 201 so that its optical axis lies on the object to be monitored (e.g. patient chest). Then, the autofocus function of the camera is activated in step 202. This causes the camera 101 to constantly adjust its focus accordingly to the movement of the monitored object. Next, the autofocus parameter pattern is monitored in step 203. In step 204 the object movement is determined on the basis of the autofocus parameter change - for example, the autofocus parameters are converted to physical dimensions, e.g. representing object position change as a measure of distance. The system analyzes the pattern in step 205. For example, the pattern can be compared with model breathing patterns stored in a database 301. A discrepancy in the measured signal pattern can then trigger an alarm signal in step 206. For example, the alarm signal can be used to wake up a sleeping person or to inform a caretaker of the sleeping/lying person about abnormal breathing. This reduces the need for attention of the caretaker towards the lying person.

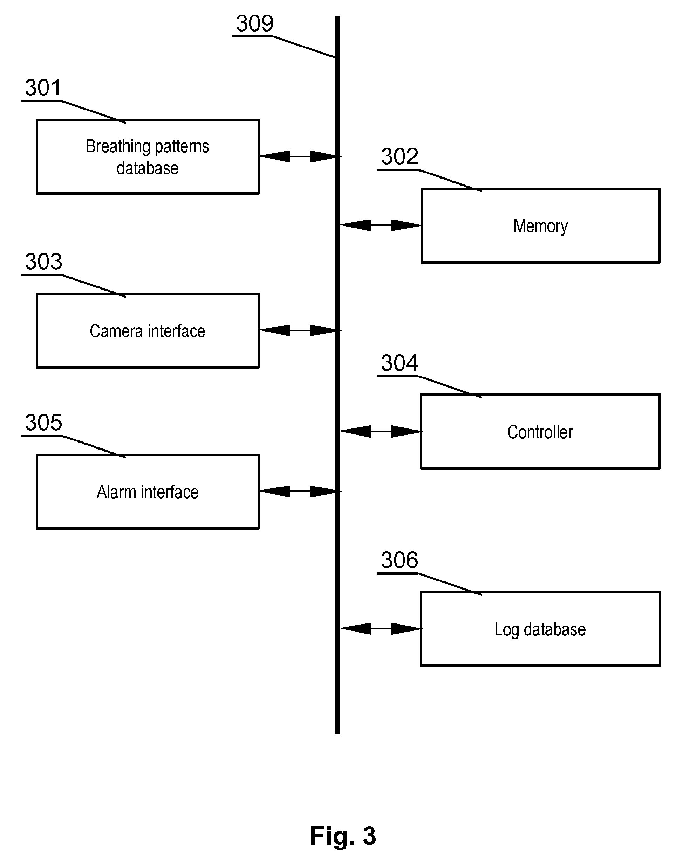

Fig. 3 shows a system for monitoring a moving object. The system comprises a model parameter patterns database 301, which stores e.g. breathing patterns used for comparison with monitored signal. It can comprise signals of normal breathing, for example of a child, a middle aged adult or an elderly person. It can also store abnormal breathing patterns, indicative of known conditions or symptoms related to health problems, so that more precise information can be provided to the user earlier. The system comprises a data bus 309 for communicating, preferably bi-directionally, all circuits of the system. Further, the system comprises a memory 302 for storing required software for the controller 304 and any temporary data needed for operation of the system. The controller 304 is configured to execute software that allows for monitoring and analyzing autofocus parameters patterns and comparing them with patterns stored in breathing patterns database 301. The controller 304 can also act to trigger appropriate actions based on detected abnormal patterns, for example an alarm signal via an alarm interface 305, which can be connected to an alarm device, e.g. a loudspeaker. The system comprises a camera interface 303 to communicate with an external camera and to collect from the camera the autofocus parameter value, which is fed to the controller 304. Logs of movement patterns can be stored in a log database 306 for future analyses.

The system may be implemented by using programmable logic circuits such as PGA (Programmable Gate Array), FPGA (Field-Programmable Gate Array) or ASIC (Application-Specific Integrated Circuit).

It can be easily recognized, by one skilled in the art, that the aforementioned system and method for monitoring of movement of object can be performed and/or controlled by one or more computer programs. Such computer programs are typically executed by utilizing the computing resources in a computing device such as personal computers, personal digital assistants, cellular telephones, receivers and decoders of digital television or the like. Applications are stored on a non-transitory medium. An example of a non-transitory medium is a non-volatile memory, for example a flash memory or volatile memory, for example RAM. The computer instructions are executed by a processor. These memories are exemplary recording media for storing computer programs comprising computer-executable instructions performing all the steps of the computer-implemented method according the technical concept presented herein.

While the invention presented herein has been depicted, described, and has been defined with reference to particular preferred embodiments, such references and examples of implementation in the foregoing specification do not imply any limitation on the invention. It will, however, be evident that various modifications and changes may be made thereto without departing from the broader scope of the technical concept. The presented preferred embodiments are exemplary only, and are not exhaustive of the scope of the technical concept presented herein.

Accordingly, the scope of protection is not limited to the preferred embodiments described in the specification, but is only limited by the claims that follow.

## Claims

1. A method for determining a movement of an object (100) in an optical axis (A) of a camera (101), comprising the steps of:
- placing (201) a camera with the optical axis directed towards the object;
- activating (202) camera continuous autofocus functionality;
- monitoring (203) autofocus parameter pattern change; and
- determining (204) the movement of the object depending on the autofocus parameter pattern change.

2. The method according to claim 1, further comprising the steps of:
- analyzing (205) the pattern of the movement of the object;
- outputting (206) an alarm signal upon recognizing an abnormal pattern (205).

3. The method according to claim 1, comprising comparing the determined parameter pattern with model parameter patterns from a database (301).

4. Use of the method according to any of claims 1-3 to monitor the breathing pattern of a person.

5. A computer program comprising program code means for performing steps (202-204) of the method according to claim 1 when said program is run on a computer.

6. A computer readable medium storing computer-executable instructions performing steps (202-204) of the method according to claim 1 when executed on a computer

7. A system for determining a movement of an object (100) in an optical axis (A) of a camera (101), comprising:
- a camera (101) placed with the optical axis (A) directed towards the object (100); and
- a computer system connected to the camera (101) via a camera interface (303) and comprising a controller (304) configured to:
- activate (202) camera continuous autofocus functionality;
- monitor (203) autofocus parameter pattern change; and
- determine (204) the movement of the object depending on the autofocus parameter pattern change.

8. The system according to claim 7, further comprising an alarm interface (305), wherein the controller (304) is configured to analyze (205) the pattern of the movement of the object and to output (206) an alarm signal via the alarm interface (305) upon recognizing an abnormal pattern (205).

9. The system according to claim 7, further comprising a database (301) of model parameter patterns, wherein the controller (304) is configured to compare the determined parameter pattern with model parameter patterns from the database (301).
